# EUROPEAN PATENT SPECIFICATION

(11) **EP 0 574 655 B1**
(45) Date of publication and mention of the grant of the patent: **08.04.1998**
(21) Application number: 93103034.0
(22) Date of filing: 26.02.1993
(51) Int. Cl.: C10M 135/36

(54) **Lubricating compositions containing aromatic amine derivatives of 2,5-dimercapto-1,3,4-thiadiazoles**
Schmiermittelzusammensetzungen, enthaltend aromatische Aminderivate von 2,5-Dimercapto-1,3,4-Thiadiazolen
Compositions lubrifiantes contenant des dérivés amino aromatique de 2,5-dimercapto-1,3,4-thiadiazoles

(30) Priority: 17.06.1992 US 899974
(43) Date of publication of application: 22.12.1993
(73) Proprietor: R.T. VANDERBILT COMPANY, Inc., Norwalk, Connecticut 06856-5150 (US)
(72) Inventor: Karol, Thomas J., Norwalk/Conn. (US); Donnelly, Steven G., New Fairfield/Conn. (US)
(74) Representative: Cohausz & Florack Patentanwälte

(56) References cited:
- EP-A- 0 275 449
- EP-A- 0 322 362
- EP-A- 0 524 452
- US-A- 2 765 289
- US-A- 4 761 482
- US-A- 4 990 273

## Description

### BACKGROUND OF THE INVENTION

The present invention concerns aromatic amine derivatives of thiadiazole compounds and their use as multifunctional additives for lubricating compositions. More particularly, the thiadiazoles are derived from 2,5-dimercapto-1,3,4-thiadiazole, -thiadiazole, an aldehyde and an aromatic amine compound.

Additives known as antiwear agents are employed to increase the load-carrying capacity of lubricants. The antiwear additives promote the formation of a surface film and thereby prevent wear of the contacting metal surfaces.

During the course of use, lubricants are susceptible to deterioration due to oxidation. The oxidative process leads to the loss of lubricating properties and inadequate protection of the device to be lubricated. Antioxidants are added to inhibit the oxidative process. Therefore, it is desirable that antiwear agents possess antioxidant properties.

U.S. Pat. No. 2,765,289 teaches reaction products of 2,5-dimercapto-1,3,4-thiadiazoles, aldehyde and diarylamine having an aldehyde-carbon to nitrogen bond. The products possess corrosion inhibiting properties.

U.S. Pat. No. 4,990,273 discloses similar reaction products having extreme pressure and antiwear properties.

U.S. Pat. No. 4,761,482 discloses the use of terpene derivatives of 2,5-dimercapto-1,3,4-thiadiazole as lubricating additives. The 2- and 5-position of said 2,5-dimercapto-1,3,4-thiadiazole are substituted with terpene groups and alkyl derivatives but not with an aromatic amine.

It has been discovered that 2,5-dimercapto-1,3,4-thiadiazole, aldehyde and diarylamines may be reacted by alkylation process yielding products having the aldehyde-carbon bonded to the aromatic ring instead to the nitrogens as in prior art. These products display antiwear and antioxidant properties when incorporated into lubricating compositions.

In accordance with the invention, there are provided improved oil-based lubricating compositions comprising a major amount of base oil and an effective amount to impart antiwear and antioxidant properties to said composition, of a 1,3,4-thiadiazole characterized by the structural formula I and II. 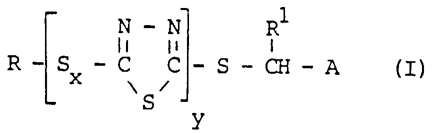 and 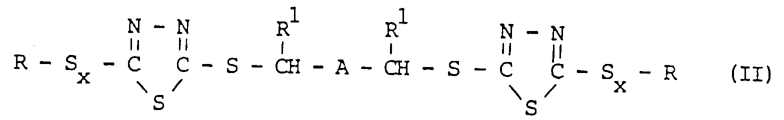 wherein x = 1 - 2, y = 1 2, R represents the group 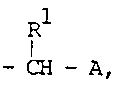 hydrogen, alkyl, cycloalkyl, aralkyl, terpene residue, a polymeric alpha-olefin residue which contains 20 to 100 carbon atoms and is unsubstituted or has a substituent hydroxy group in the 2-position, and a succinate residue of the formula 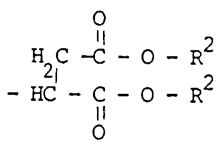 R¹ represents hydrogen, C₁₋₁₇ - alkyl, phenyl and phenyl substituted by alkyl groups; A represents aromatic ring structure selected from the group consisting of diphenylamine, phenylenediamine, N,N- or N,N'-diphenyl--p-phenylenediamine, N-phenyl-p-phenylenediamine, naphthylamine, phenyl-1-naphthylamine, phenyl-2-naphthylamine, quinoline, hydrated quinoline and phenothiazine and wherein the aromatic ring and amine groups may be substituted by alkyl groups; and R² represents hydrogen, alkyl and cycloalkyl groups; provided R in formula (I) is not hydrogen when A is diphenylamine, phenothiazine, alkyl-substituted diphenylamine or alkyl-substituted phenothiazine and provided that A is bonded at a carbon atom of the ring.

A further aspect of the invention concerns a method for protection of metal surfaces from wear by applying improved lubricating oil compositions, the improvement of which consists of adding to the composition an effective amount of a 1,3,4-thiadiazole compound characterized by the structural formula I and II.

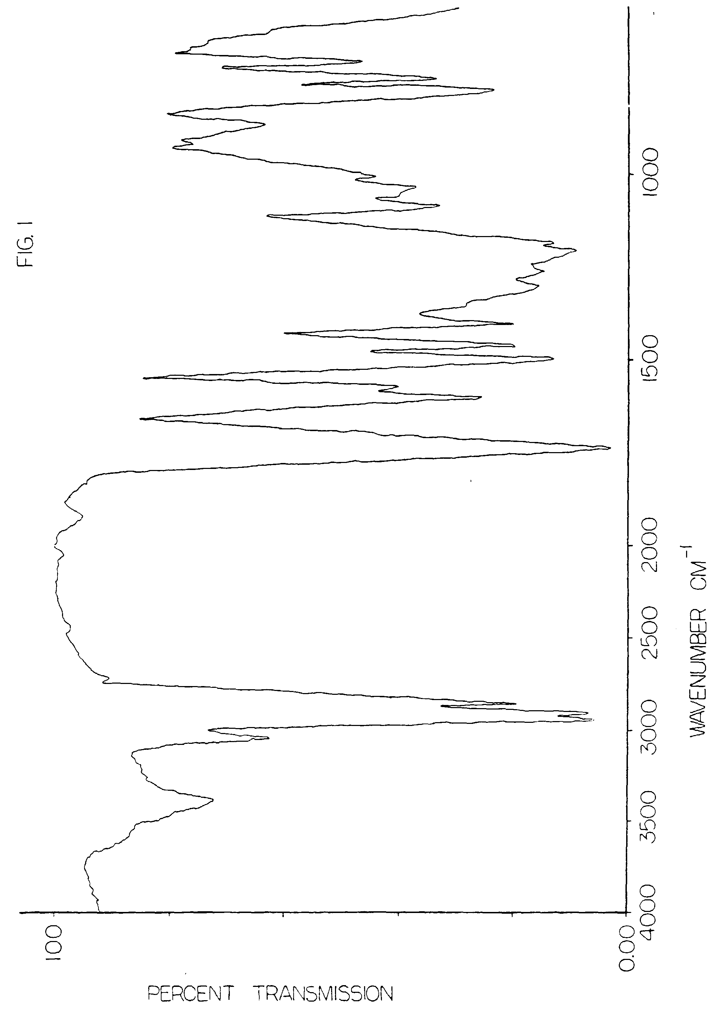

Figure 1 shows infrared spectrum of the present reaction product of 2-(1,2-di(2-ethylhexoxycarbonyl)ethylthio)-1,3,4-thiadiazole, paraformaldehyde and phenyl-1-naphthylamine and further described in Example 3 hereinbelow. The absorption at 3384 cm⁻¹ indicates N - H bond stretching absorption maximum.

### DESCRIPTION OF SPECIFIC EMBODIMENTS

The compounds of the invention may be prepared by reacting 2,5-dimercapto-1,3,4-thiadiazole, aldehyde and an aromatic amine by an alkylation process. Preferred are reaction products wherein the thiadiazole, aldehyde and aromatic amine ranges in the molar ratio of 1:1:1 to 2:4:3.

The reaction is essentially an alkylation process wherein the 2,5-dimercapto-1,3,4-thiadiazole and aldehyde form a hydroxy-intermediate which through dehydration attaches to a carbon on the ring of the aromatic amine. The position of attachment to the ring may vary and mixtures may be formed. The products are characterized by aromatic amine N-H bond stretching absorption in the region of 3175 to 3450 cm⁻¹.

The reaction may be conducted in the presence or absence of a suitable inert solvent, such as toluene, dimethyl ether and others. Optionally, the reaction may employ acid catalysts. For example, a Lewis acid catalyst such as methanesulfonic acid may be used.

The aldehyde reactant may be a normal or branched chain aliphatic aldehyde containing 1 to 18 carbon atoms or an aromatic aldehyde. Examples of suitable aldehydes include, among others, formaldehyde, acetaldehyde, benzaldehyde, 2-ethylhexyl aldehyde, butyraldehyde, caprylic aldehyde, phenylacetaldehyde, and salicylaldehyde.

The aromatic amine may be selected from aromatic monoamines and diamines. The aromatic compounds may be substituted by alkyl groups on the amine group and the aromatic ring. The alkyl groups may be normal or branched chain. Particularly preferred are alkyl groups having 1 to 18 carbon atoms. Specific compounds include, among others, diphenylamine, alkylated diphenylamine such as N,N'-di-sec-butyl-p-phenylenediamine, 4,4'-dioctyldiphenylamine, p-phenylene-diamine, alkylated phenylenediamine, N,N'-dioctylphenylenediamine, N-(1,3-dimethylbutyl)-N'-phenyl-p-phenylenediamine, phenothiazine, phenyl-1-naphthylamine, phenyl-2-naphthylamine, 1,2-dihydro-2,2,4-trimethyl- quinoline and its polymers.

The novel 1,3,4-thiadiazole derivatives may be substituted in the 5-position by alkyl, cycloalkyl, aryl and aralkyl groups, terpene residues, polymeric alpha-olefin residues and succinate residues. The alkyl groups may be straight or branched chain and may contain up to 100 carbon atoms. Representative alkyl groups, among others, include methyl, butyl, 2-ethylhexyl, dodecyl, and octadecyl groups. Exemplary cycloalkyl groups include cyclopentyl, cyclohexyl and cycloheptyl. Preferred aryl groups are phenyl, naphthyl, phenothiazinyl and quinolyl groups. Particularly preferred terpene residues are pinene residue of the formula 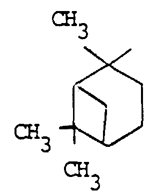 and limonene residue of the formula

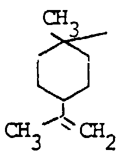

The polymeric alpha-olefin residue is essentially a hydrocarbyl radical having 20 to 200 carbon atoms. Typically, the molecular weight of the polymeric residue ranges from 280 to 2600 and higher. The polymers may have straight or branched chain aliphatic units having 2 to 10 carbon atoms. Especially useful are polymers and copolymers of alpha-olefins as for example isoprene, isobutene, 2-methyl-1-heptene, ethylene, propylene, and 2-methyl-5-propylhexene. The polymeric alpha-olefin residue may be derived from a hydrocarbon polymer with an epoxide or chlorine functionality. These activated polyolefins are available commercially. Activated polyisobutenes with epoxide functionality are marketed under the trade name ACTIPOL™ by Amoco Chemical Company. Alternately, commercial polyolefins may be epoxidized by known methods.

The succinate residue in the above formulae may be derived from maleic anhydride or acid and further esterified with normal and branched chain alkyl groups containing 1 to 22 carbon atoms and cyclic aliphatic groups such as cyclohexyl, cyclopentyl and cycloheptyl.

The thiadiazole derivatives of the invention are useful as additives for lubricants. The compounds possess multifunctional properties. In addition to being effective antiwear agents, they also perform oxidation inhibiting functions.

The lubricating compositions contemplated herein include lubricating oils and lubricating greases containing a major amount of base oil. The base oil may be selected from naphthenic, aromatic, paraffinic, mineral and synthetic oils. The synthetic oils may be selected from, among others, alkylene polymers, polysiloxanes, carboxylic acid esters and polyglycol ethers.

The amount of the thiadiazole additive required to be effective for imparting antiwear and antioxidant characteristics to lubricating compositions may range from about 0.01 to 15.0 percent of the lubricating composition. The preferred range is about 0.1 to 5.0 percent of the additive based on the weight of the lubricating composition.

The lubricating compositions may contain the necessary ingredients to formulate the composition, as for example emulsifiers, dispersants and viscosity improvers. Greases may be prepared by adding thickeners, as for example, salts and complexes of fatty acids, polyurea compounds, clays and quaternary ammonium bentonite complexes. Depending on the intended use of the lubricant, other functional additives may be added to enhance a particular property of the lubricant. The lubricating compositions may further contain extreme pressure agents, metal passivators, rust inhibitors, dispersants and other known antioxidants and antiwear agents.

The following examples are given for the purpose of further illustrating the invention. All percentages and parts are based on weight unless otherwise indicated.

### Example 1

### 2-Pinanyl-5-(4,4'-dioctylphenylamino-(o or m)-phenylene)methylenethio-1,3,4-thiadiazole.

A reactor was charged with alpha-pinene, 49.0 g (0.36 moles), 2,5-dimercapto-1,3,4-thiadiazole, 53.3 g (0.36 moles), and toluene, 50 ml. The reaction mixture was cautiously heated to 130 - 140° C, followed by addition of 91% paraformaldehyde, 12.2 g (0.37 moles). After heating for one hour, the reactor was charged with p,p'-dioctyldiphenylamine, 141.5 g (0.36 moles), and toluene, 50 ml. Water was azeotroped off at about 135° C. The product was stripped and filtered.

### Example 2

### 4,4'-Bis(2,2'-di(2-hydroxy polyolefin)thio)-1,3,4-thiadiazole-5-yl-thiomethylene diphenylamine.

A reactor was charged with 2-(2-hydroxy polyolefin)thio-1,3,4-thiadiazole-5-thiol, 178.2 g (0.242 moles), petroleum distillate solvent, 76 g, and paraformaldehyde, 9.3 g (0.31 moles), and heated to 130° C. Diphenylamine, 20.4 g (0.121 moles), was charged to the reactor and water was azeotroped off at about 130° C. The product was filtered and stripped.

### Example 3

### Reaction product of 2-(1,2-di(2-ethylhexoxycarbonyl)ethylthio)-1,3,4-thiadiazole, paraformaldehyde and phenyl-1-naphthylamine.

A reactor was charged with 2-(1,2-di(2-ethylhexoxycarbonyl)ethylthio)-1,3,4-thiadiazole, 1400 g, and 91% paraformaldehyde, 110 g and heated at about 130-135° C for about 0.5 hours. The intermediate, 160.4 g, and phenyl-1-naphthylamine, 69.2 g, and toluene, 100 ml was added and water was azeotroped off at about 135° C. The product was stripped and filtered. The infrared spectrum showing N - H bond stretching absorption maximum at 3384 cm⁻¹ is presented in Fig. 1.

### Example 4

### 2- (Phenothiazinylisobutylenethio)-1,3,4-thiadiazole-5-thione.

A reactor was charged with 2,5-dimercapto-1,3,4-thiadiazole, -thiadiazole, 15 g, in 20 ml dioxane and isobutyraldehyde, 7.5 g. The temperature was increased to 43° C and 20 g of phenothiazine was added. The reaction was heated to reflux and maintained at about 115° C for 1.5 hours. Water was then azeotroped off using hexane. The reaction was cooled to room temperature. The product was recrystallized from acetone.

### Example 5

### Reaction product of 2-pinanyl-1,3,4-thiadiazole-5-thiol, isobutyraldehyde and diphenylamine.

A reactor was charged with alpha-pinene, 75 g, 2,5-dimercapto-1,3,4-thiadiazole, 75 g, and rinsed with acetone, 3 ml. The reaction was heated cautiously to 130 - 135° C and then maintained at that temperature for 5 minutes. The reaction was stripped of excess pinene and acetone with aspirator under reduced pressure. After cooling to 50^{o} C, the reaction was charged with isobutyraldehyde, 40 g, and diphenylamine, 83.5 g. The reactor was fitted with Dean Stark attachment filled with hexane and heated to 130° C. After collecting water, the hexane volume was adjusted for a reflux at 130^{o} C and the reaction was azeotroped for 8 hours. The product was stripped and filtered. Infrared spectrum presented in Fig. 1 showed good N - H bond absorption in the region of 3324 to 3392 cm⁻¹.

### Example 6

### Reaction product of 2-hydroxymethylthio-1,3,4-thiadiazole-5-thiol and diphenylamine.

A reactor was charged with 2,5-dimercapto-1,3,4-thiadiazole, 30 g, and 100 ml dioxane and mixed. After addition of 37% formaldehyde, 16.3 g, water was azeotroped. Diphenylamine, 33.85 g, was added and the mixture was heated using hexane to azeotrope water. After cooling to room temperature, the mixture was dried over magnesium sulfate, filtered and the solvent was stripped off under vacuum at 110 to 115^{o}C.

### Example 7

### Thin Film Oxygen Uptake Test.

A test was conducted essentially according to the method described by Chia-Soon Ku et al, J. Am. Soc. Lubricating Eng., 40, 2,75-83, 1984. The oxidation induction time of the lubricant was measured under conditions which simulate the high temperature oxidation process in automotive engines by a modified rotary bomb oxidation test method ASTM D-2272. The test was conducted with 1.5 gram samples of a base motor oil (SAE 30,SF). The oil was fully formulated with the exception of the antioxidant additive. The test was conducted at 160^{o} C and initial oxygen pressure of 620.6 kPa (90 psi). A "pass" oil has a high induction time, while a "fail" oil has a low induction time. Compounds of the invention were added to the oil in the amount indicated in Table I. The data indicate that the additives of the invention have good antioxidant properties.

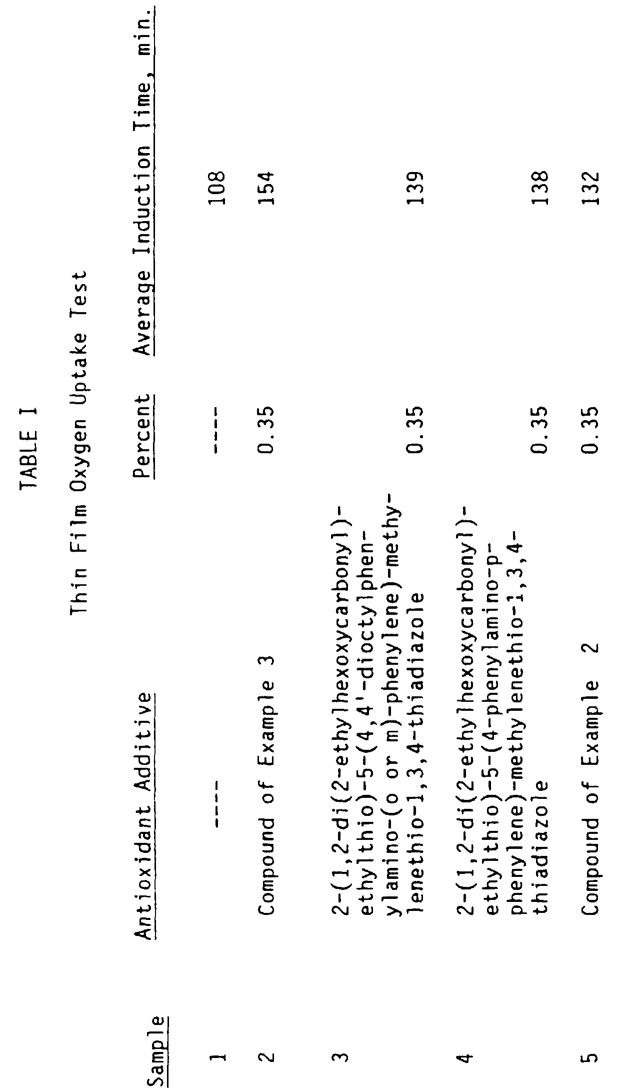

### Example 8

### Modified Falex Wear Test.

A laboratory test was conducted by using the original Falex machine to simulate the valve train wear of an automobile engine. The V-blocks and pin were washed in mineral spirits with an ultrasonic cleaner, rinsed with acetone, air-dried and weighed. The test sample (60 g) was placed into the oil cup. The motor was switched on and the loading arm was placed on the ratchet wheel. Upon reaching the reference load of 227 kg, the ratchet wheel was disengaged and the load was maintained constant for 3.5 hours. Thereafter, the motor was switched off. The V-blocks and pin were washed, dried and weighed. The weight loss, a measure of wear, was recorded and compiled in Table II.

The test samples were prepared by adding the compounds of the invention to the base motor oil (SAE 30, SF) in the amount given in Table II. The base oil contained 0.11 percent phosphorus and no supplemental antioxidant. The results indicate that the present compounds afford good antiwear properties.

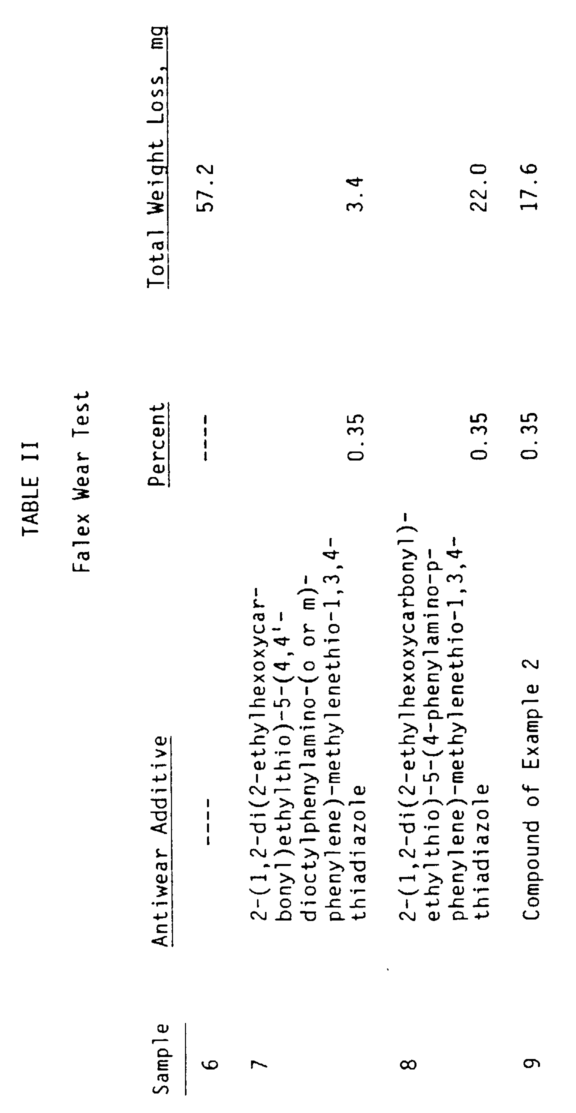

The above embodiments have shown various aspects of the present invention. Other variations will be evident to those skilled in the art and such modifications are intended to be within the scope of the invention as defined by the appended claims.

## Claims

1. A lubricating composition comprising a major portion of an oil of lubricating viscosity and a minor wear and oxidation inhibiting amount of a compound selected from the group consisting of compounds having the structural formulae 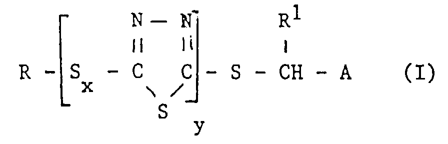 and 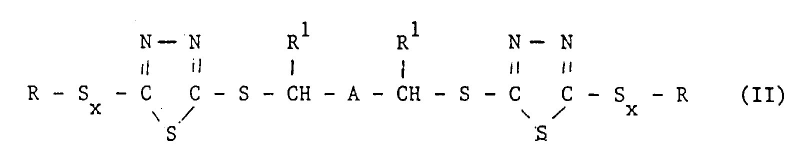 wherein x = 1 - 2, y = 1 - 2, R represents the group 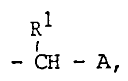 hydrogen, alkyl, cycloalkyl, aralkyl, terpene residue, a polymeric alpha-olefin residue which contains 20-100 carbon atoms and is unsubstituted or has a substituent hydroxy group in the 2-position, and a succinate residue of the formula 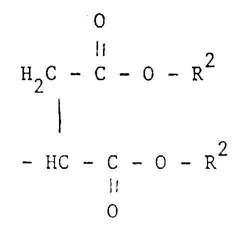 R¹ represents hydrogen, C₁₋₁₇-alkyl, phenyl and phenyl substituted by alkyl groups; A represents aromatic ring of compounds selected from the group consisting of diphenylamine, phenylenediamine, N,N- or N,N -diphenyl-p-phenylenediamine, N-phenyl-p-phenylenediamine, naphthylamine, phenyl-1-naphthylamine, phenyl-2-naphthylamine, quinoline, hydrated quinoline and phenothiazine and wherein the aromatic ring and amine groups may be substituted by alkyl groups; and R² represents hydrogen, alkyl and cycloalkyl groups; provided R in formula (I) is not hydrogen when A is diphenylamine, phenothiazine, alkyl-substituted diphenylamine or alkyl-substituted phenothiazine and provided that A is bonded at a carbon atom of the ring.

2. A lubricating composition according to claim 1 which contains a thickener.

3. A method for protecting metal surfaces from wear which comprises contacting the surfaces with a lubricating composition comprising a major amount of base oil of lubricating viscosity, the improvement of which consists of adding to the oil a minor wear and oxidation inhibiting amount of an additive selected from the group of compounds having the structural formulae 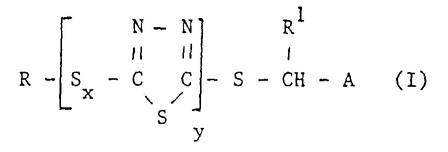 and 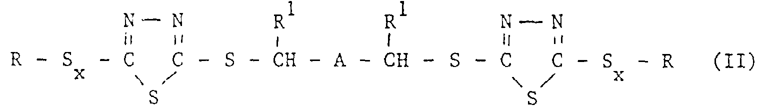 wherein x = 1 - 2, y = 1 - 2, R represents the group 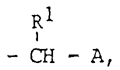 hydrogen, alkyl, cycloalkyl, aralkyl, terpene residue, a polymeric alpha-olefin residue which contains 20-100 carbon atoms and is unsubstituted or has a substituent hydroxy group in the 2-position, and a succinate residue of the formula 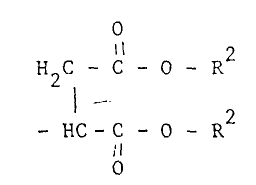 R¹ represents hydrogen, C₁₋₁₇- alkyl, phenyl and phenyl substituted by alkyl groups; A represents aromatic ring of compounds selected from the group consisting of diphenylamine, phenylenediamine, N,N- or N,N'-diphenyl-p-phenylenediamine, N-phenyl-p-phenylenediamine, naphthylamine, phenyl-1-naphthylamine, phenyl-2-naphthylamine, quinoline, hydrated quinoline and phenothiazine and wherein the aromatic ring and amine groups may be substituted by alkyl groups; and R² represents hydrogen, alkyl and cycloalkyl groups; provided R in formula (I) is not hydrogen when A is diphenylamine, phenothiazine, alkyl-substituted diphenylamine or alkyl-substituted phenothiazine and provided that A is bonded at a carbon atom of the ring.

## Patentansprüche

1. Schmiermittel, enthaltend einen Hauptanteil eines Öls mit Schmierviskosität und eine geringe, den Verschleiß und die Oxidation hemmende Menge einer Verbindung, die ausgewählt ist aus der Gruppe der Verbindungen mit der Strukturformel 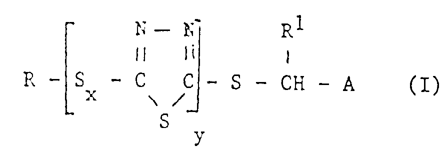 und 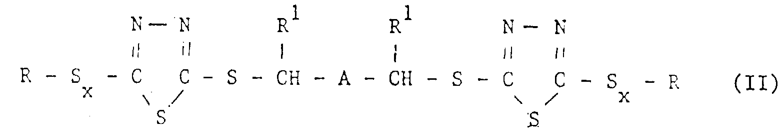 worin x = 1 bis 2, y = 1 bis 2 ist, R die Gruppe CHR¹-A, Wasserstoff, Alkyl, Cycloalkyl, Aralkyl, Terpenrest, einen polymeren Alphaolefinrest, der 20 bis 100 Kohlenstoffatome enthält und unsubstituiert ist oder eine Hydroxygruppe als Substituenten in der 2-Stellung enthält, und einen Succinatrest der Formel 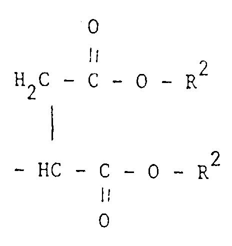 bedeutet; R¹ bedeutet Wasserstoff, C₁₋₁₇-Alkyl, Phenyl und durch Alkylgruppen substituiertes Phenyl; A bedeutet einen aromatischen Ring, der ausgewält ist aus Diphenylamin, Phenylendiamin, N,N- oder N,N'-Diphenyl-p-phenylendiamin, N-Phenyl-p-phenylendiamin, Naphthylamin, Phenyl-l-naphthylamin, Phenyl-2-naphthylamin, Quinolin, hydratisiertes Quinolin und Phenothiazin und worin der aromatische Ring und Amingruppen durch Alkylgruppen substituiert sein können; R² bedeutet Wasserstoff, Alkyl und Cycloalkylgruppen; vorausgesetzt, daß R in Formel (I) nicht Wasserstoff ist, wenn A Diphenylamin, Phenothiazin, alkyl-substituiertes Diphenylamin oder alkyl-substituiertes Phenothiazin ist und vorausgesetzt, daß A an ein Kohlenstoffatom im Ring gebunden ist.

2. Schmiermittel nach Anspruch 1, das ein Verdickungsmittel enthält.

3. Verfahren zum Schutz von Metalloberflächen vor Verschleiß, welches das Inkontaktbringen der Oberflächen mit einem Schmiermittel umfaßt, welches einen Hauptanteil eines Grundöls mit Schmierviskosität enthält, wobei die Verbesserung darin besteht, daß dem Öl eine geringe, den Verschleiß und die Oxidation hemmende Menge einer Verbindung zugesetzt ist, die ausgewält ist aus der Gruppe der Verbindungen mit der Strukturformel 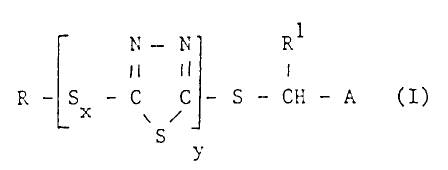 und 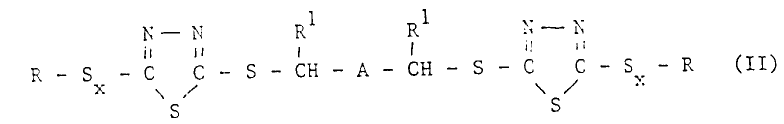 worin x = 1 bis 2, y = 1 bis 2 ist, R die Gruppe CHR¹-A, Wasserstoff, Alkyl, Cacloalkyl, Aralkyl, Terpenrest, einen polymeren Alphaolefinrest, der 20 bis 100 Kohlenstoffatome enthält und unsubstituiert ist oder eine Hydroxygruppe als Substituenten in der 2-Stellung enthält, und einen Succinatrest der Formel 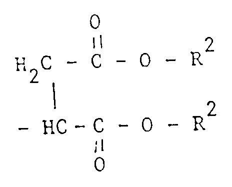 bedeutet; R¹ bedeutet Wasserstoff, C₁₋₁₇-Alkyl, Phenyl und durch Alkylgruppen substituiertes Phenyl; A bedeutet einen aromatischen Ring, der ausgewält ist aus Diphenylamine, Phenylendiamin, N,N- oder N,N'-Diphenyl-p-phenylendiamin, N-Phenyl-p-phenylendiamin, Naphthylamin, Phenyl-1-naphthylamin, Phenyl-2-naphthylamin, Quinolin, hydratisiertes Quinolin und Phenothiazin und worin der aromatische Ring und Amingruppen durch Alkylgruppen substituiert sein können; R² bedeutet Wasserstoff, Alkyl und Cycloalkylgruppen; vorausgesetzt, daß R in Formel (I) nicht Wasserstoff ist, wenn A Diphenylamin, Phenothiazin, alkyl-substituiertes Diphenylamin oder alkyl-substituiertes Phenothiazin ist und vorausgesetzt, daß A an ein Kohlenstoffatom im Ring gebunden ist.

## Revendications

1. Composition lubrifiante comprenant une quantité majoritaire d'une huile de viscosité lubrifiante et une quantité minoritaire d'un composé inhibiteur de l'usure et de l'oxydation choisi dans le groupe formé par les composés ayant la formule de structure 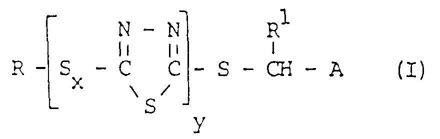 et 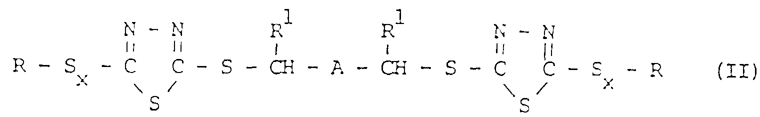 dans lesquelles x = 1 - 2, y = 1 - 2, R représente le groupe -C(R¹)H-A, un atome d'hydrogène, un groupe alkyle, cycloalkyle, aralkyle, un radical terpène, un radical polymèrique alpha-oléfinique contenant de 20 à 100 atomes de carbone et étant non substitué ou substitué en position 2- par un groupe hydroxyl, et un radical succinate de formule 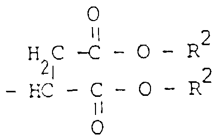 R1 représente un atome d'hydrogène, un groupe alkyle en C1-C17, un groupe phényle ou phényle substitué par des groupes alkyle; A représente une structure à cycle aromatique choisie dans le groupe formé par la diphénylamine, la phénylènediamine, la N,N- ou N,N'-diphényl-p-phényldiamine, la N-phényl-p-phénylènediamine, la naphtylamine, la phényl-1-naphtylamine, la phényl-2-naphtylamine, la quinoline, la quinoline hydratée et la phénothiazine et dans laquelle le cycle aromatique et les groupes amine peuvent être substitués par des groupes alkyle; et R2 représente l'hydrogène, un groupe alkyle, et cycloalkyle; sous la condition que R dans la formule (I) ne soit pas l'hydrogène quand A est diphényldiamine, phénothiazine, diphénylamine alkyl-substitué ou phénothiazine alkyl-substitué et pourvu que A soit lié à un atome de carbone du cycle.

2. Composition lubrifiante selon la revendication 1 qui contient un épaississant.

3. Procédé de protection de surfaces de métal contre l'usure comprenant la mise en contact d'une composition lubrifiante comprenant une quantité majoritaire d'une huile de base de viscosité lubrifiante, l'amélioration consistant en une addition à l'huile d'une quantité minoritaire d'un additif inhibiteur d'usure et d'oxydation choisi dans le groupe de composés ayant les formules de structure 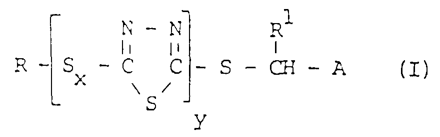 et 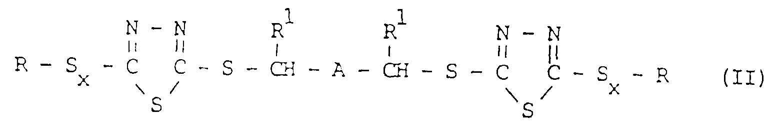 dans lesquelles x = 1 - 2, y = 1 - 2, R représente le groupe -C(R¹)H-A, un atome d'hydrogène, un groupe alkyle, cycloalkyle, aralkyle, un radical terpène, un radical polymérique alpha-oléfinique contenant de 20 à 100 atomes de carbone et étant non substitué ou substitué en position 2- par un groupe hydroxyl, et un radical succinate de formule 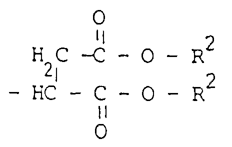 R1 représente un atome d'hydrogène, un groupe alkyle en C1-C17, un groupe phényle ou phényle substitué par des groupes alkyle; A représente une structure à cycle aromatique choisie dans le groupe formé par la diphénylamine, la phénylènediamine, la N,N- ou N,N'-diphényl-p-phényldiamine, la N-phényl-p-phénylènediamine, la naphtylamine, la phényl-1-naphtylamine , la phényl-2-naphtylamine, la quinoline, la quinoline hydratée et la phénothiazine et dans laquelle le cycle aromatique et les groupes amine peuvent être substitués par des groupes alkyle; et R2 représente l'hydrogène, un groupe alkyle, et cycloalkyle; sous la condition que R dans la formule (I) ne soit pas l'hydrogène quand A est diphényldiamine, phénothiazine, diphénylamine alkyl-substitué ou phénothiazine alkyl-substitué et pourvu que A soit lié à un atome de carbone du cycle.
